# EUROPÄISCHE PATENTSCHRIFT

(11) **EP 1 946 604 B1**
(45) Veröffentlichungstag und Bekanntmachung des Hinweises auf die Patenterteilung: **25.02.2015**
(21) Anmeldenummer: 06806650.5
(22) Anmeldetag: 01.11.2006
(51) Int. Cl.: H04R 1/10, A61F 11/14, H04R 5/033, H04R 7/12

(54) **Kopfhörer und Headset mit einer aktiven Lärmkompensation**
Headphone and headset with an active noise compensation apparatus
Écouteurs et casque micro avec un système actif de compensation du bruit

(30) Priorität: 02.11.2005 DE 102005052548
(43) Veröffentlichungstag der Anmeldung: 23.07.2008
(73) Patentinhaber: Sennheiser electronic GmbH & Co. KG, 30900 Wedemark (DE)
(72) Erfinder: GRELL, Axel, 31303 Burgdorf (DE); GRANDT, André, 30900 Wedemark (DE)
(74) Vertreter: Eisenführ Speiser
(86) Internationale Anmeldenummer: PCT/EP2006/010491
(87) Internationale Veröffentlichungsnummer: WO 2007/051606

(56) Entgegenhaltungen:
- EP-A2- 0 967 592
- WO-A2-01/56330
- US-A- 5 182 774

## Beschreibung

Die vorliegende Erfindung betrifft ein elektroakustisches Wandlersystem für eine aktive Lärmkompensationsvorrichtung sowie ein Headset mit einem derartigen Wandlersystem.

Herkömmliche aktive Lärmkompensationsvorrichtungen weisen ein Wiedergabewandlersystem mit einem Sensormikrofon auf, welches zwischen dem elektroakustischen Wandler und dem Ohr eines Trägers, insbesondere möglichst nah am Ohr des Trägers, angeordnet ist. Der von dem Sensormikrofon aufgezeichnete Schall wird zur aktiven Lärmkompensation verwendet.

DE 1 876 054 zeigt eine Membran für einen elektroakustischen Wandler, wobei ein Teil der Membran kalottenförmig gewölbt ist.

US 5,182,774 zeigt ein Headset mit einer aktiven Lärmkompensationsvorrichtung. Hierbei ist ein Sensormikrofon zwischen einem Wandlersystem und dem Ohr eines Trägers angeordnet, so dass das Mikrofon mechanisch hervorsteht und eine gewisse Verletzungsgefahr vorhanden sein kann.

Es ist somit Aufgabe der vorliegenden Erfindung, ein Wandlersystem und/oder eine aktive Lärmkompensationsvorrichtung vorzusehen, welche eine verringerte Verletzungsgefahr aufweist.

Diese Aufgabe wird durch ein Wandlersystem für eine aktive Lärmkompensationsvorrichtung gemäß Anspruch 1 sowie durch ein Headset gemäß Anspruch 6 gelöst

Somit wird ein Wandlersystem für eine aktive Lärmkompensationsvorrichtung vorgesehen, welches ein erstes öhrseitiges Ende und einen elektroakustischen Wiedergabewandler aufweist. Der elektroakustische Wandler weist ein Membransystem und ein Magnetsystem auf, wobei der zentrale Bereich des Membransystems zum Magnetsystem hin gewölbt ist und als inverse Kalotte ausgebildet ist. Ein Sensormikrofon ist in dem Bereich der Wölbung des Membransystems angeordnet.

Die Erfindung bezieht sich auf ein Wandlersystem für eine aktive Lärmkompensationsvorrichtung, wobei ein (elektro)akustischer Wandler eine inverse Kalotte aufweist und das Sensormikrofon vom Ohr aus betrachtet vor dem elektroakustischen Wandler bzw. dem Membransystem angeordnet ist, d. h. das Sensormikrofon ist axial beabstandet zum Membransystem angeordnet. Dies hat insbesondere den Vorteil, dass die Bauhöhe einer aktiven Lärmkompensationsvorrichtung verringert wird und eine Verletzungsgefahr reduziert wird. Das Sensormikrofon wird somit in dem Bereich zwischen der inversen Kalotte und einem Ohr eines Trägers angeordnet. Durch eine derartige Anordnung kann das Sensormikrofon sehr nahe an dem Ohr eines Trägers angeordnet werden. Dies ist insbesondere vorteilhaft für die aktive Lärmkompensation. Die Kalotte des akustischen Wändlers ist hierbei derart ausgestaltet, dass sie zum (axial beabstandeten) Magnetsystem hin gewölbt ist. Somit kann das Sensormikrofon innerhalb des durch die Anordnung der inversen Kalotte entstandenen freien Volumens, das durch die Innenwölbung der Membran entstanden ist, platziert werden.

Weitere Ausgestaltungen der Erfindung sind Gegenstand der Unteransprüche.

Nachfolgend wird die Erfindung unter Bezugnahme auf die beigefügte Zeichnung näher erläutert.

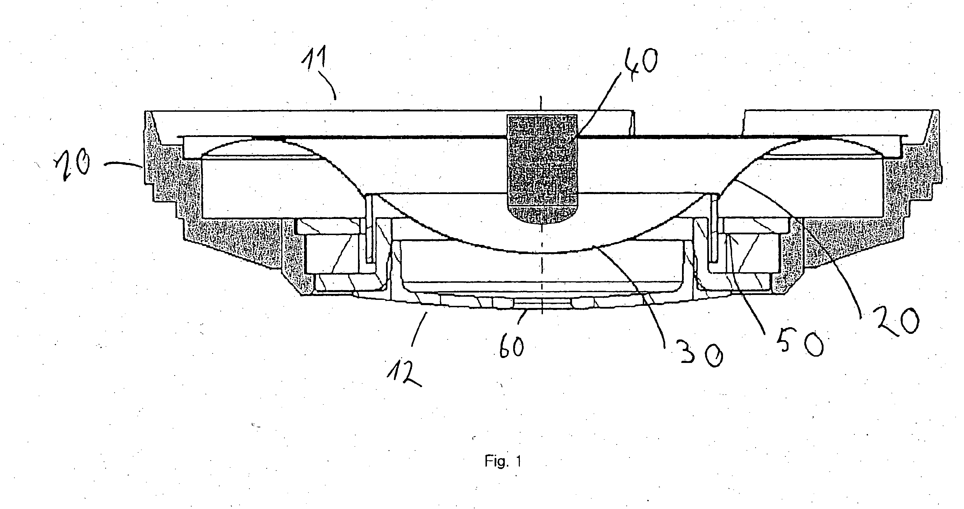

- Fig. 1: zeigt eine schematische Schnittansicht eines elektroakustischen Wiedergabewandlers gemäß der Erfindung.

Fig. 1 zeigt eine schematische Schnittansicht eines elektroakustischen Wandlersystems. Insbesondere ist hier ein elektroakustischer Wiedergabewandler 10 zum Wiedergeben von Audiosignalen gezeigt, welcher beispielsweise in einer aktiven Lärmkompensationsvorrichtung eingesetzt werden kann. Der elektroakustische Wandler 10 weist ein erstes ohrseitiges Ende 11 und ein zweites ohrabgewandtes Ende 12 sowie ein Membransystem 20 mit einer inversen Kalotte 30 auf. In dem freien Volumen, welches durch die Innenwölbung des Membransystems durch die Anordnung der inversen Kalotte 30 entstanden ist (d. h. dem Volumen zwischen der inversen Kalotte 30 und dem ersten Ende 11 bzw. dem Ohr des Trägers), ist ein Sensormikrofon 40 zum Aufzeichnen von Lärm bzw. Schall vorgesehen, welches für eine aktive Lärmsdhallkompensation benötigt wird. Der Wandler 10 weist ferner ein Magnetsystem 50 sowie eine von einem Ohr abgewandte Öffnung 60 im zweiten Ende 12 auf.

Eine aktive Lärmkompensationsvorrichtung ist in der Patentschrift EP 0 737 022 B1 gezeigt. Insbesondere wird in den Fig. 1 bis 3 sowie in den Absätzen [0019] bis [0028] das Prinzip der aktiven Lärmkompensationsvorrichtung mit einem Wiedergabewandler und einem Sensormikrofon beschrieben. Die Kompensationsschaltung ist in der Fig. 3 und im Absatz [0028] gezeigt.

Die Löcher bzw. Öffnungen 60 im zweiten ohrabgewandten Ende dienen dazu, dass die sich in dem Volumen zwischen der inversen Kalotte 30 und dem Gehäuse 10 befindliche Luft nach außen weichen kann. Vorzugsweise sollte der Abstand zwischen dem Sensormikrofon 40 und der inversen Kalotte 30 möglichst gering sein, ohne dass das Mikrofon 40 und die inverse Kalotte bei maximaler Auslenkung zusammenstoßen. Durch einen möglichst geringen Abstand zwischen dem Sensormikrofon 40 und der inversen Kalotte 30 wird die Laufzeit verringert, so dass die für die aktive Lärmkompensation benötigte 180° Phasenverschiebung des aufgenommenen Schalls besser erreichbar ist. Der Wiedergabewandler dient dazu, das lärmkompensierte Audiosignal wiederzugeben, wobei die Lärmkompensation wie in der EP 0 737 022 B1, Fig. 3, Absatz [0028] beschrieben anhand des durch das Sensormikrofon aufgezeichneten Schalls bzw. Lärms erfolgt.

Somit wird das Sensormikrofon 40 direkt vor der inversen Kalotte 30 angeordnet. Das Sensormikrofon 40 wird dabei vorzugsweise durch einen Resonator (nicht gezeigt) gehalten,

Durch die in Fig. 1 beschriebene Anordnung der inversen Kalotte 30 und des Sensormikrofons 40 kann die Übertragungsstrecke zwischen dem elektroakustischen Wandler 10 und dem Sensormikrofon bzw. dem Messmikrofori 40 für die aktive Lärmschallkompensation erheblich reduziert werden.

Obwohl in Fig. 1 eine bezüglich eines Ohres im Wesentlichen senkrechte Ausrichtung des Sensormikrofons 40 gezeigt ist, kann das Sensormikrofon auch in einem beliebigen anderen Winkel angeordnet werden.

Das Magnetsystem 50 ist vorzugsweise wie in Fig. gezeigt außen angeordnet, so dass die inverse Kalotte 30 dazwischen Platz findet. Alternativ dazu kann das Magnetsystem auch innen angeordnet sein. In einem derartigen Fall sind die Magnete jedoch bevorzugt entsprechend der Wölbung der inversen Kalotte 30 geformt. Die Formung des Magnetsystems kann zu akustischen Dimensionierung verwendet werden.

## Patentansprüche

1. Kopfhörer, mit
einer aktiven Lärmkompensationsvorrichtung, die einen elektroakustischen Wiedergabewandler (10) und ein Sensormikrofon (40) zum Aufnehmen von Störschall aufweist,
wobei der elektroakustische Vhiedergabewandler (10) ein erstes ohrseitiges Ende (11), ein Membransystem (20) und ein Magnetsystem (50) aufweist, **dadurch gekennzeichnet, dass**
ein zentraler Bereich (30) des Membransystems (20) zum Magnetsystem (50) hin gewölbt ist und als inverse Kalotte (30) ausgebildet ist und
dass das Sensormikrofon (40) in einem Volumen zwischen dem ersten Ende (11) und der inversen Kalotte (30) des Membransystems (20) angeordnet ist und sich in den Bereich der inversen Kalotte (30) hinein erstreckt.

2. Kopfhörer nach Anspruch 1, wobei
die Richtwirkung des Sensormikrofons (40) zum ersten Ende (11) des Wandlersystems ausgerichtet ist.

3. Kopfhörer nach Anspruch 1 oder 2, wobei
der Abstand zwischen dem Sensormikrofon (40) und der inversen Kalotte (30) derart ausgestaltet wird, dass sich das Sensormikrofon (40) und die inverse Kalotte (30) auch bei einer maximalen Auslenkung der inversen Kalotte (30) nicht berühren.

4. Headset, mit
einer aktiven Lärmkompensationsvorrichtung, die einen elektroakustischen Wiedergabewandler (10) und ein Sensormikrofon (40) zum Aufnehmern von Störschall aufweist,
wobei der elektroakustische Wiedergabewandler (10) ein erstes ohrseitiges Ende (11), ein Membransystem (20) und ein Magnetsystem (50) aufweist, **dadurch gekennzeichnet, dass**
ein zentraler Bereich (30) des Membransystems (20) zum Magnetsystem (50) hin gewölbt ist und als inverse Kalotte (30) ausgebildet ist und
dass das Sensormikrofon (40) in einem Volumen zwischen dem ersten Ende (11) und der inversen Kalotte (30) des Membransystems (20) angeordnet ist und sich in den Bereich der inversen Kalotte (30) hinein erstreckt.

## Claims

1. Headset, having
an active noise compensation device which has an electroacoustic reproduction converter (10) and a sensor microphone (40) for recording ambient noise,
the electroacoustic reproduction converter (10) having a first ear-side end (11), a membrane system (20) and a magnet system (50), **characterised in that**
a central region (30) of the membrane system (20) is curved in the direction towards the magnet system (50) and is constructed as an inverse spherical cap (30) and
**in that** the sensor microphone (40) is arranged in a space between the first end (11) and the inverse spherical cap (30) of the membrane system (20) and extends into the region of the inverse spherical cap (30).

2. Headset according to claim 1, wherein
the directive efficiency of the sensor microphone (40) is orientated towards the first end (11) of the converter system.

3. Headset according to claim 1 or claim 2, wherein
the spacing between the sensor microphone (40) and the inverse spherical cap (30) is configured in such a manner that the sensor microphone (40) and the inverse spherical cap (30) do not touch each other even in the event of maximum redirection of the inverse spherical cap (30).

4. Headset, having
an active noise compensation device which has an electroacoustic reproduction converter (10) and a sensor microphone (40) for recording ambient noise,
the electroacoustic reproduction converter (10) having a first ear-side end (11), a membrane system (20) and a magnet system (50), **characterised in that**
a central region (30) of the membrane system (20) is curved in the direction towards the magnet system (50) and is constructed as an inverse spherical cap (30) and
**in that** the sensor microphone (40) is arranged in a space between the first end (11) and the inverse spherical cap (30) of the membrane system (20) and extends into the region of the inverse spherical cap (30).

## Revendications

1. Casque d'écoute, avec
un dispositif actif de compensation de bruit qui comprend un transducteur électroacoustique de reproduction (10) et un microphone détecteur (40) pour l'enregistrement de bruits parasites,
le transducteur électroacoustique de reproduction (10) comprenant une première extrémité (11) côté oreille, un système de membrane (20) et un système d'aimant (50), **caractérisés**
**en ce qu'**un secteur central (30) du système de membrane (20) est bombé vers le système d'aimant (50) et est conçu en forme de calotte inversée (30) et
**en ce que** le microphone détecteur (40) est disposé dans un volume entre la première extrémité (11) et la calotte inversée (30) du système de membrane (20) et s'étend jusque dans le secteur de la calotte inversée (30) .

2. Casque d'écoute selon la revendication 1,
l'effet directif du microphone détecteur (40) étant orienté vers la première extrémité (11) du système transducteur.

3. Casque d'écoute selon la revendication 1 ou 2,
la distance entre le microphone détecteur (40) et la calotte inversée (30) étant agencée de manière telle que le microphone détecteur (40) et la calotte inversée (30) ne viennent pas en contact même lors d'une excursion maximale de la calotte inversée (30).

4. Casque-micro avec
un dispositif actif de compensation de bruit qui comprend un transducteur électroacoustique de reproduction (10) et un microphone détecteur (40) pour l'enregistrement de bruits parasites,
le transducteur électroacoustique de reproduction (10) comprenant une première extrémité (11) côté oreille, un système de membrane (20) et un système d'aimant (50), **caractérisé**
**en ce qu'**un secteur central (30) du système de membrane (20) est bombé vers le système d'aimant (50) et est conçu en tant que calotte inversée (30) et
**en ce que** le microphone détecteur (40) est disposé dans un volume entre la première extrémité (11) et la calotte inversée (30) du système de membrane (20) et s'étend jusque dans le secteur de la calotte inversée (30).
